# EUROPÄISCHE PATENTSCHRIFT

(11) **EP 0 701 731 B1**
(45) Veröffentlichungstag und Bekanntmachung des Hinweises auf die Patenterteilung: **03.04.2002**
(21) Anmeldenummer: 94919596.0
(22) Anmeldetag: 01.06.1994
(51) Int. Cl.: G10K 11/30

(54) **VORRICHTUNG ZUR BEHANDLUNG VON BIOLOGISCHEM GEWEBE UND KÖRPERKONKREMENTEN**
DEVICE FOR THE TREATMENT OF BIOLOGICAL TISSUE AND CONCRETIONS IN THE BODY
DISPOSITIF POUR LE TRAITEMENT DE TISSUS BIOLOGIQUES ET DE CONCRETIONS CORPORELLES

(30) Priorität: 01.06.1993 DE 4318237
(43) Veröffentlichungstag der Anmeldung: 20.03.1996
(73) Patentinhaber: STORZ MEDICAL AG, CH-8280 Kreuzlingen (CH)
(72) Erfinder: WESS, Othmar, CH-8574 Lengwill-Oberhofen (CH)
(74) Vertreter: Münich, Wilhelm, Dr.
(86) Internationale Anmeldenummer: EP9401784
(87) Internationale Veröffentlichungsnummer: WO9428540

(56) Entgegenhaltungen:
- EP-A- 0 369 177
- EP-A- 0 434 931
- CH-A- 284 836
- DE-A- 2 919 176
- DE-A- 3 040 635
- DE-A- 3 727 692
- US-A- 4 562 900

## Beschreibung

### Technisches Gebiet

Die Erfindung bezieht sich auf eine Vorrichtung zur Behandlung von biologischem Gewebe und zur Zerstörung von Konkrementen mit fokussierten akustischen Wellen gemäß dem Oberbegriff des Patentanspruchs 1.

### Stand der Technik

Im Zuge der Entwicklung nichtinvasiver Therapieverfahren sind Methoden erarbeitet worden, die eine eng lokalisierte, gezielte Wirkung im Körperinneren erzeugen, ohne daß es erforderlich wäre, die Kärperhülle traumatisch zu durchdringen. Ein Beispiel hierfür ist die extrakorporal induzierte Lithotripsie, bei der kurze Druckimpulse hoher Amplitude außerhalb des Körpers erzeugt werden, die auf das zu zerstörende Konkrement, wie beispielsweise eine Nierenstein fokussiert sind. Ist die Energiedichte am Ort des Konkrements ausreichend hoch, so wird das Konkrement zertrümmert. Ein Beispiel für eine derartige Vorrichtung ist in der DE 38 35 318 C1 angegeben, auf die sowie auf die in dieser Druckschrift genannte Literatur ausdrücklich zur Erläuterung aller hier nicht näher erläuterten Begriffe Bezug genommen wird. Weiterhin ist vorgeschlagen worden, die mit Hilfe hochenergetischer Ultraschallwellen konzentrierte Energie zur Hyperthermie oder zur Thermotherapie einzusetzen.

Bei allen Verfahren, bei denen fokussierte akustische Wellen zur Erzielung eines Therapieerfolges verwendet werden, ist es erforderlich, die außerhalb des Körpers erzeugte Energie unter Ausnutzung geeigneter "Körper-Fenster" in den Körper einzukoppeln und mittels geeigneter Ortungsverfahren auf das vorbestimmte Zielgebiet zu lenken. Hierbei ist zu berücksichtigen, daß viele Organe nicht direkt über geeignete "Körper-Fenster" erreichbar sind. Die zur Verfügung stehenden Körper-Fenster sind vielmehr unregelmäßig geformt, wie beispielsweise die Fenster, durch die Ultraschall in die Leber oder Harnblase eingekoppelt werden kann. Darüberhinaus ist es häufig erforderlich, den Ultraschall unter einer "schiefen" bzw. geneigten Einfallsrichtung einzustrahlen.

Die bislang vorgeschlagenen Schallerzeugungseinheiten, wie sie beispielsweise in der DE 38 35 318 C1 genannt sind, also beispielsweise sphärische Kalotten, Hohl-elipsoide oder Rotationsparaboloide sind damit nur eingeschränkt einsetzbar, da die bei diesen Schallerzeugungseinheiten vorhandenen kreisförmigen Abstrahlflächen nicht optimal an die vorhandenen Körper-Fenster angepaßt sind, und darüberhinaus bei einer schiefwinkligen Einkoppelungsrichtung sich die Koppelmedien nur schlecht an die Körperkonturen anpassen können.

Dies hat zur Folge, daß nur ein Teil der außerhalb des Körpers erzeugten Ultraschallenergie am Zielort für therapeutische Zwecke zur Verfügung steht. Darüberhinaus ist die örtliche und zeitliche Fokussierung aufgrund von Phasenstörungen auf der Ausbreitungsstrecke (Koppelmedium und Körpergewebe) häufig nur unzureichend. Hinzu kommt, daß nur ein gewisser Teil der erzeugten Ultraschallenergie in das Koppelmedium abgegeben wird, da insbesondere bei Verwendung von piezoelektrischen Erregern die akustischen Impedanzen (Dichte 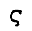 * Ausbreitungsgeschwindigkeit c) der Schallerzeuger und des gewebeähnlichen Übertragungs- bzw. Koppelmediums (häufig Wasser) sehr unterschiedlich sind, so daß nur eine teilweise Energieeinkopplung möglich ist.

Der Erfindung liegt die Aufgabe zugrunde, eine Vorrichtung zur Behandlung von biologischem Gewebe und zur Zerstörung von Konkrementen mittels fokussierter akustischer Wellen anzugeben, die aufgrund ihres Aufbaus leicht an die verschiedenen Einsatzfälle anpaßbar ist, und die darüber hinaus nicht auf kreisförmige Abstrahlflächen beschränkt ist. Eine erfindungsgemäße Lösung dieser Aufgabe ist im Patentanspruch 1 angegeben. Weiterbildungen der Erfindung sind Gegenstand der Ansprüche 2ff.

Erfindungsgemäß weist die Schallerzeugungseinheit in an sich bekannter Weise eine Ebene oder nur gering -konkav oder konvex - gekrümmte Abstrahlfläche auf, so daß die erzeugte Welle eine im wesentlichen ebene Welle ist. Zur Fokussierung dieser im wesentlichen ebenen Welle hat die in einer festen räumlichen Beziehung zur Schallerzeugungseinheit angeordnete Fokussiereinheit auf ihrer Schalleintritts- und/oder Schallaustrittsfläche eine Fresnellinsen-artige Struktur. Fresnellinsen sind nicht nur aus der Lichtoptik bekannt, sondern auch für Ultraschallabbildungssysteme bereits beispielsweise in der DE 29 19 176 C2 vorgeschlagen worden. Derartige Fresnellinsen sind so aufgebaut, daß kohärenter Ultraschall an den einzelnen Stufen der Linse eine Phasendifferenz von n * λ aufweist (n = 0,1,2...), so daß die Ultraschallwellen, die sich durch benachbarte Zonen der Linse ausbreiten, beim Austritt aus der Linse konstruktiv in der Fokuszone interferieren.

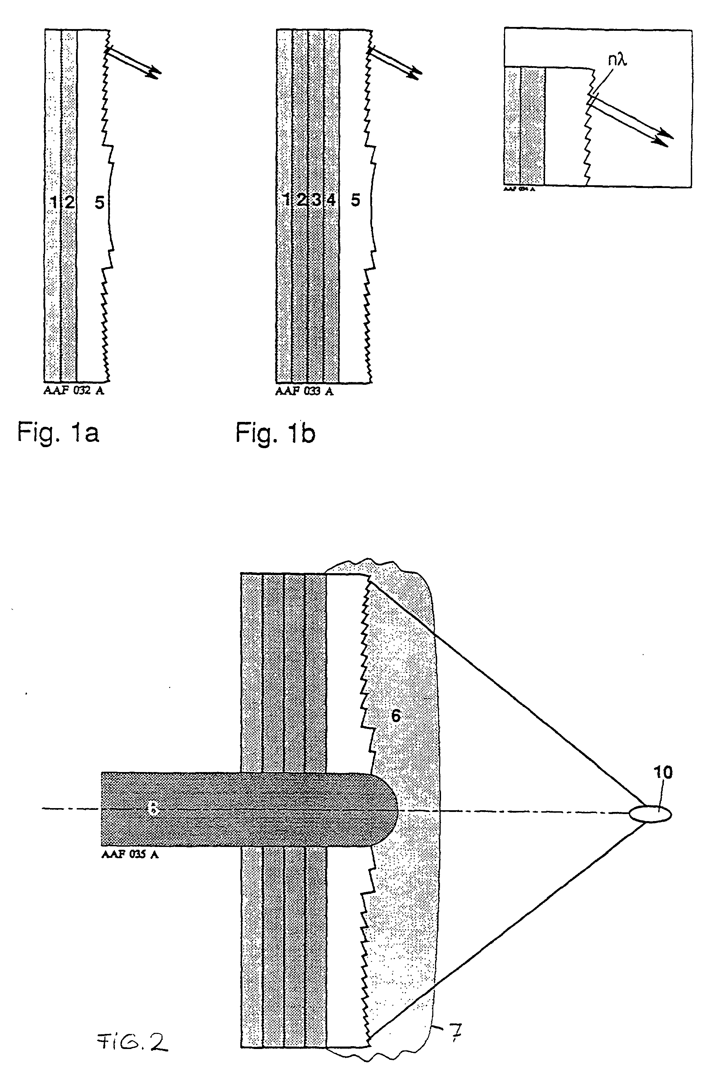

Der Grundgedanke der Erfindung wird anhand der Figur 1 erläutert. Ein geeignetes piezoelektrisches Material 2 (z. B. Ba-Titanat) befindet sich in engem Kontakt mit einer Anpasschicht 1 (z. B. Messing o.ä.) wahlweise mit ähnlicher Impedanz wie das piezoelektrische Material oder mit sehr unterschiedlicher Impedanz (z.B. Luft, zur Vermeidung der Energieabstrahlung nach hinten) und ist nach vorne, zur Applikationsseite hin, mit einer Fresnellinse 5 verbunden wie sie oben, oder in dem Patent DE 29 19 176 beschrieben ist. Vorteilhaft ist die Verwendung eines Linsenmaterials, das in der Impedanz zwischen der Piezokeramik und der des Koppelme-diums liegt. Als Materialien kommen z. B. Aluminium oder auch Polystyrol in Betracht. Vorteilhaft ist auch die Verwendung einer oder mehrerer Anpassschichten 3,4, die eine reflexionsarme Impedanzanpassung vom Erzeugungsmaterial (z.B. Piezokeramik) zum Koppelmedium (z.B. Wasser) und schließlich zum Körpergewebe hin ermöglichen. Die Fresnellinse selbst kann dabei als tragende Struktur für den gesamten Verbundschwinger dienen, wodurch sich ein geringes Gesamtgewicht und eine gute Handhabung ergibt. Wie in Figur 2 dargestellt, kann ein solcher Fresnelverbundschwinger mit einer geeigneten Ortungseinrichtung 8 zur Darstellung und Kontrolle des Zielgebietes 10, kombiniert werden, ebenso wie mit einer variablen Vorlaufstrecke 6 zur Anpassung an unterschiedliche anatomische Gegebenheiten.

Durch Verwendung geeigneter Koppelmedien 6 (z. B. Wasser, US-Koppelgel, Gelscheiben o.ä.) können die anatomisch bedingten Abstände zwischen den Applikatoren und der Körperoberfläche angepaßt werden. Das Koppelmedium 6 kann z. B: durch ein flexibles Kissen 7 begrenzt werden.

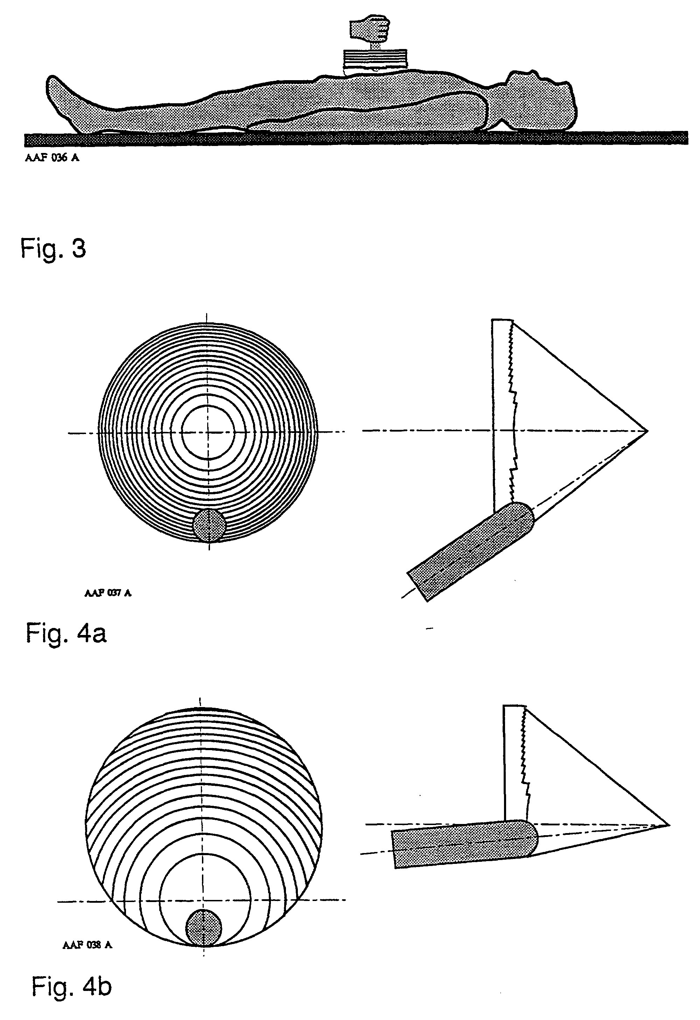

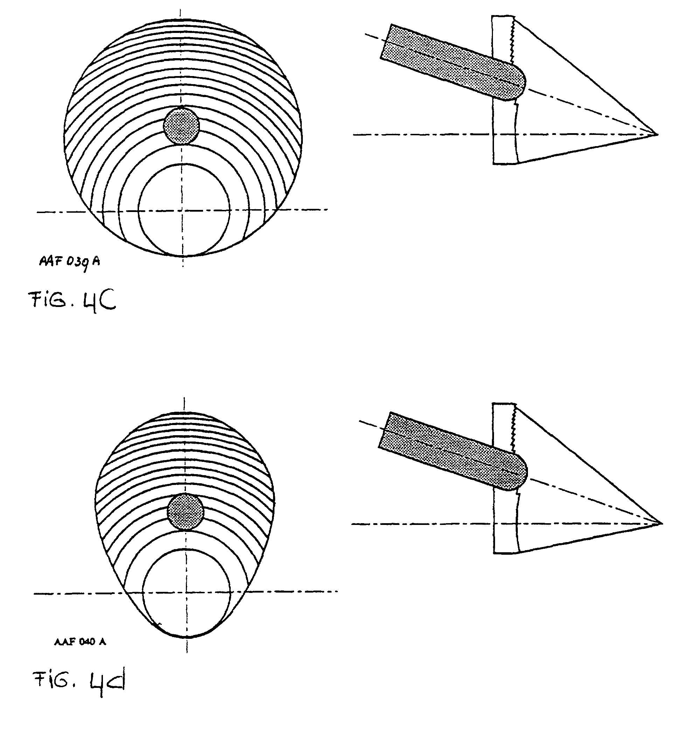

Figur 3 zeigt eine mögliche Anwendung eines derartigen Applikators an einem Patienten.

Durch die flache Bauform des Applikators läßt er sich leicht auf dem Körper des Patienten ansetzen. Der diagnostische Ultraschall erlaubt das Auffinden des Zielgebietes, wobei die Handhabung ähnlich der eines diagnostische Ultraschall - B-Bild-Schwingers am Patienten ist.

Je nach anatomischen Gegebenheiten lassen sich neben Applikatoren mit kreisförmigen Außenmaßen auch solche mit beliebigen Formen verwirklichen, wobei auch die Lage und Orientierung des Ortungskomponente variieren kann. Einige Beispiele sind in Figur 4a-d dargestellt. Die in Figur 4c dargestellte Anordnung, eignet sich z.B. zur Behandlung von Prostataanomalien durch die gefüllte Harnblase hindurch mit nach unten abgewinkelter Einstrahlrichtung. Weitere geeignete Applikatoren, die den speziellen Fenstern und Einstrahlrichtungen angepaßt sind, lassen sich in nahezu beliebiger Weise festlegen. Man kann so die Energieeinkopplung optimieren und den speziellen anatomischen Gegebenheiten anpassen, ohne an die sonst gerätebedingten kreisförmigen Koppelfenster gebunden zu sein.

Ein weiterer Vorteil der Erfindung besteht daran, daß man unter Verwendung breitbandiger Schwinger durch Variation der Anregungsfrequenz den Fokusabstand zum Applikator verändern kann. So ist es möglich, durch Erhöhung der Ultraschallfrequenz den Fokus näher am Applikator zu erzeugen und durch Erniedrigung der Frequenz ihn weiter herauszuschieben.

Dieser "Zoom-Effekt" erlaubt es, ohne variable Verlaufstrecke zu therapieren. Hieraus ergeben sich Vorteile bezüglich verlustfreier Einkopplung und geringerer Phasenstörungen. Durch eine bessere Konzentration der Energie ergeben sich besser lokalisierte und wirkungsvollere Fokus- und Therapiezonen.

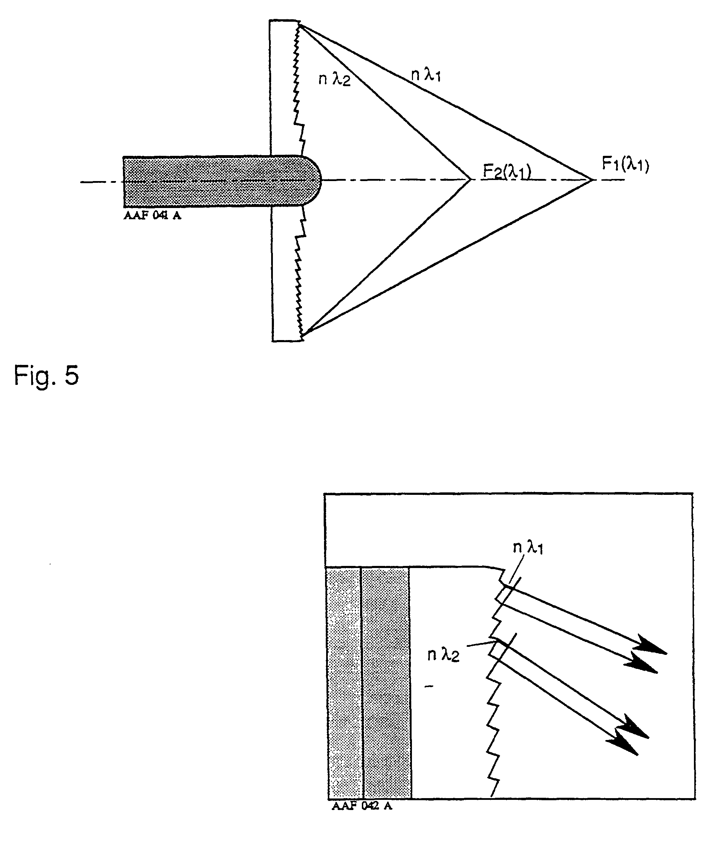

Wie in Figur 5 dargestellt, lassen sich von der Körperoberfläche aus in direktem Kontakt des Applikators mit der Haut verschiedene Tiefen der Körperzonen erreichen.

## Patentansprüche

1. Vorrichtung zur Behandlung von biologischem Gewebe und zur Zerstörung von Konkrementen mittels fokussierter akustischer Wellen, mit
- einer Schallerzeugungseinheit, die eine ebene oder nur gering gekrümmte Abstrahlfläche aufweist, so dass die erzeugte akustische Welle eine ebene Welle ist,
- einer Steuereinheit, die die Schallerzeugungseinheit derart ansteuert, daß die erzeugte ebene akustische Welle eine bestimmte Wellenlänge λ und Energie hat, und
- einer Fokussiereinheit, die in einer festen räumlichen Beziehung zur Schallerzeugungseinheit steht, so dass sie die erzeugten ebenen Wellen in einen Fokusbereich fokussiert, in dem die Energiedichte zur Behandlung von biologischem Gewebe und zur Zerstörung von Konkrementen ausreicht,
- wobei die Fokussiereinheit zur Fokussierung der akustischen Wellen in dem Fokusbereich eine Fresnellinsen-artige Struktur aufweist, die stufig ausgebildet ist,
**dadurch gekennzeichnet, dass**
die Stufenhöhe der Fresnellinsen-artigen Struktur so gewählt ist, dass sie für Wellen, die sich von der Schallaustrittsseite her in Richtung Fokusbereich ausbreiten, ein ganzzahliges Vielfaches einer typischen akustischen Wellenlänge λ beträgt, und
dass entweder die von der Kreisform abweichende Form der Abstrahlfläche der Fokussiereinheit und/oder die Form des Abstrahlkegels der Fokussiereinheit und damit die Lage des Fokus bezogen auf die Abstrahlfläche der Fokussiereinheit dem jeweiligen Einsatzfall angepaßt ist,
und/oder dass die Schallerzeugungseinheit wenigstens einen breitbandigen Schwinger aufweist, dessen Anregungsfrequenz die Steuereinheit zur Einstellung des Abstandes des Fokusbereichs von der Fokussiereinheit variiert.

2. Vorrichtung nach Anspruch 1,
**dadurch gekennzeichnet, daß** die Fokussiereinheit aus einem Material besteht, dessen akustische Impedanz zwischen der Impedanz der Schallerzeugungseinheit und der Impedanz des Mediums im Schallweg nach der Fokussiereinheit liegt.

3. Vorrichtung nach Anspruch 2,
**dadurch gekennzeichnet, daß** die zwischen der Abstrahlfläche der Schallerzeugungseinheit und der Schalleintrittsfläche der Fokussiereinheit vorgesehene(n) Anpaßschicht(en) eine Impedanz hat (haben), die zwischen der der Fokussiereinheit und der Impedanz der Schallerzeugungseinheit liegen.

4. Vorrichtung nach Anspruch 3,
**dadurch gekennzeichnet, daß** die Anpaßschichten reflexionsarm sind.

5. Vorrichtung nach Anspruch 3 oder 4,
**dadurch gekennzeichnet, daß** die Anpaßschichten wenigstens teilweise λ/4-Schichten sind.

6. Vorrichtung nach einem der Ansprüche 1 bis 5,
**dadurch gekennzeichnet, daß** auf der der Abstrahlfläche gegenüberliegenden Begrenzungsfläche der Schallerzeugungseinheit mindestens eine Anpaßschicht aufgebracht ist.

7. Vorrichtung nach Anspruch 6,
**dadurch gekennzeichnet, daß** die auf der gegenüberliegenden Begrenzungsfläche aufgebrachte Anpaßschicht entweder eine ähnliche Impedanz wie die Schallerzeugungseinheit oder eine stark unterschiedliche Impedanz hat.

8. Vorrichtung nach einem der Ansprüche 1 bis 7,
**dadurch gekennzeichnet, daß** die Schallerzeugungseinheit Piezoschwinger (2) aufweist.

9. Vorrichtung nach einem der Ansprüche 1 bis 8,
**dadurch gekennzeichnet, daß** die Fokussiereinheit als Tragekörper für den gesamten Aufbau dient.

10. Vorrichtung nach einem der Ansprüche 1 bis 9,
**dadurch gekennzeichnet, daß** die Schallerzeugungseinheit eine Mehrzahl von Einzelschwingern aufweist.

11. Vorrichtung nach Anspruch 10,
**dadurch gekennzeichnet, daß** die Einzelschwinger mosaikartig angeordnet sind.

12. Vorrichtung nach Anspruch 10,
**dadurch gekennzeichnet, daß** die Einzelschwinger die Form von konzentrisch angeordneten Ringen haben.

13. Vorrichtung nach Anspruch 12,
**dadurch gekennzeichnet, daß** die Ringe nach Art von Fresnelzonen aufgebaut sind.

14. Vorrichtung nach einem der Ansprüche 10 bis 13,
**dadurch gekennzeichnet, daß** die Steuereinheit die Einzelschwinger getrennt ansteuert.

15. Vorrichtung nach Anspruch 14,
**dadurch gekennzeichnet, daß** die Steuereinheit die Einzelschwinger mit unterschiedlicher Phasenlage ansteuert.

16. Vorrichtung nach Anspruch 14 oder 15,
**dadurch gekennzeichnet, daß** die Steuereinheit die Einzelschwinger mit unterschiedlicher Frequenz ansteuert.

17. Vorrichtung nach einem der Ansprüche 1 bis 16,
**dadurch gekennzeichnet, daß** eine Ortungseinrichtung vorgesehen ist, die die Erfassung des zu behandelnden Bereichs und insbesondere von Steinen, Konkrementen etc. ermöglicht.

18. Vorrichtung nach Anspruch 17,
**dadurch gekennzeichnet, daß** die Ortungseinrichtung im zentralen Bereich der Schallaustrittsfläche der Fokussiereinheit angeordnet ist.

19. Vorrichtung nach Anspruch 17,
**dadurch gekennzeichnet, daß** die Ortungseinrichtung außerhalb des zentralen Bereichs der Schallaustrittsfläche der Fokussiereinheit angeordnet ist.

20. Vorrichtung nach einem der Ansprüche 17 bis 19,
**dadurch gekennzeichnet, daß** die Ortungseinrichtung eine Röntgenortungseinrichtung aufweist.

21. Vorrichtung nach einem der Ansprüche 17 bis 20,
**dadurch gekennzeichnet, daß** die Ortungseinrichtung eine Ultraschallortungseinrichtung aufweist.

22. Vorrichtung nach Anspruch 21,
**dadurch gekennzeichnet, daß** die Ultraschallortungseinrichtung ein Ultraschall-B-Bildgerät ist.

23. Vorrichtung nach einem der Ansprüche 1 bis 22,
**dadurch gekennzeichnet, daß** die Schallaustrittsfläche der fresnellinsenartigen Struktur eine rotationssymmetrische Form hat.

24. Vorrichtung nach einem der Ansprüche 1 bis 23,
**dadurch gekennzeichnet, daß** die Steuereinheit zur Variation der Fokuslage die Frequenz variiert, mit der sie die Schallerzeugungseinheit ansteuert.

25. Vorrichtung nach einem der Ansprüche 1 bis 24,
**dadurch gekennzeichnet, daß** zwischen Schallaustrittsfläche der Fokussiereinheit und dem Einkoppelbereich des zu behandelnden Körpers ein Koppelmedium vorgesehen ist.

26. Vorrichtung nach Anspruch 25,
**dadurch gekennzeichnet, daß** das Koppelmedium durch ein flexibles Kissen begrenzt ist.

27. Vorrichtung nach Anspruch 25 oder 26,
**dadurch gekennzeichnet, daß** das Koppelmedium eine flexible Vorlaufstrecke bildet.

## Claims

1. Device for treating biologic tissue and for crushing concrements by means of focussed acoustic waves, comprising
- a sound generator unit having a planar emission surface or an emission surface with only a slight curvature so that the generated acoustic wave is a planar wave,
- a controller unit controlling said sound generator unit in such a way that the generated planar acoustic wave will have a defined wave length λ and a defined energy, and
- a focussing unit in a fixed three-dimensional relationship with said sound generator unit so that it will focus the generated planar waves into a focussing range where the energy density is sufficient for the treatment of biologic tissue and for crushing concrements,
- with said focussing unit including a structure similar to a Fresnel lens and having a multi-stage configuration for focussing the acoustic waves into said focussing range,
**characterised in that**
the height of the stages of said structure resembling a Fresnel lens is so selected that it corresponds to an integer multiple of a typical acoustic wave length λ for waves propagating from the sound output side towards the focussing range, and
that either the shape of the emission surface of said focussing unit, which varies from the circular shape, and/or the shape of the emission cone of said focussing unit and hence the focus position relative to the emission surface of said focussing unit is matched with the respective application,
and/or that said sounds generator unit comprises at least one wide-band vibrator whose excitation frequency varies said controller unit for adjusting the distance of said focus range from said focussing unit.

2. Device according to Claim 1,
**characterised in that** said focussing unit consists of a material whose acoustic impedance is in the range between the impedance of said sound generator unit and the impedance of the medium, along the sound propagation path downstream of said focussing unit.

3. Device according to Claim 2,
**characterised in that** the matching layer(s) provided between the emission surface of said sound generator unit and the sound entry surface of said focussing unit has/have an impedance level between that of said focussing unit and the impedance of said sound generator unit.

4. Device according to Claim 3,
**characterised in that** said matching layers are low-reflection layers.

5. Device according to Claim 3 or 4,
**characterised in that** said matching layers are λ/4 layers at least in part.

6. Device according to any of the Claims 1 to 5,
**characterised in that** at least one matching layer is applied on the limiting surface of said sound generator unit, which is opposite to the emission surface.

7. Device according to Claim 6,
**characterised in that** said matching layer applied on the opposite limiting surface has either an impedance similar to that of said sounds generator unit or a distinctly different impedance.

8. Device according to any of the Claims 1 to 7,
**characterised in that** said sound generator unit comprises piezo vibrators (2).

9. Device according to any of the Claims 1 to 6,
**characterised in that** said focussing unit serves as body supporting the entire structure.

10. Device according to any of the Claims 1 to 9,
**characterised in that** said sounds generator unit comprises a plurality of individual vibrators.

11. Device according to Claim 10,
**characterised in that** said individual vibrators are disposed in the manner of a mosaic.

12. Device according to Claim 11,
**characterised in that** said individual vibrators have the shape of concentrically disposed rings.

13. Device according to Claim 12,
**characterised in that** said rings are configured in the manner of Fresnel zones.

14. Device according to any of the Claims 10 to 13,
**characterised in that** said controller unit controls said individual vibrators separately.

15. Device according to Claim 14,
**characterised in that** said controller unit controls said individual vibrators with different phase positions.

16. Device according to Claim 14 or 15,
**characterised in that** said controller unit controls said individual vibrators with different frequencies.

17. Device according to any of the Claims 1 to 16,
**characterised in that** a locating means is provided which permits the detection of the zone requiring treatment and particularly the sites of calculi, concrements, etc.

18. Device according to Claim 17,
**characterised in that** said locating means is arranged in the central zone of the sound output surface of said focussing unit.

19. Device according to Claim 17,
**characterised in that** said locating means is arranged outside of the central zone of the sound output surface of said focussing unit.

20. Device according to any of the Claims 17 to 19,
**characterised in that** said locating means comprises an X-ray locating means.

21. Device according to any of the Claims 17 to 20,
**characterised in that** said locating means comprises an ultrasonic locating means.

22. Device according to Claim 21,
**characterised in that** said ultrasonic locating means is an ultrasonic B-imaging device.

23. Device according to any of the Claims 1 to 22,
**characterised in that** said sound emission surface of said Fresnel-type structure presents a rotationally symmetrical shape.

24. Device according to any of the Claims 1 to 23,
**characterised in that** said controller unit varies the frequency by which it controls said sound generator unit, for varying the focus position.

25. Device according to any of the Claims 1 to 24,
**characterised in that** a coupling medium is provided between the sound emission surface of said focussing unit and the coupling zone of the body to be treated.

26. Device according to Claim 25,
**characterised in that** said coupling medium is limited by a flexible cushion.

27. Device according to Claim 25 or 26,
**characterised in that** said coupling medium constitutes a flexible advancing path.

## Revendications

1. Dispositif de traitement des tissus biologiques et à broyer des concrétions moyennant des ondes acoustiques focalisées, comprenant
- une unité génératrice de son à une surface d'émission planaire ou une surface d'émission à une courbure minime seulement, de façon, que l'onde acoustique ainsi engendrée soit une onde plane,
- une unité de commande, qui commande ladite unité génératrice de son d'une manière, que l'onde acoustique plane ainsi engendrée ait une longueur d'onde λ définie et une énergie définie, et
- une unité de focalisation en un arrangement fixe à trois dimensions relatives à ladite unité génératrice de façon, qu'elle focalise les ondes planes engendrées dans une zone de focalisation, où la densité d'énergie suffit à traiter du tissu biologique et à broyer des concrétions, ladite unité de focalisation renfermant une structure similaire à une lentille du type Fresnel, configurée à plusieurs étages pour la focalisation des ondes acoustiques dans ladite zone de focalisation,
**caractérisé en ce que**
la hauteur des étages de ladite structure, qui est similaire à une lentille du type Fresnel, est choisie de façon, qu'elle corresponde à un multiple en nombre entier d'une longueur acoustique typique λ pour des ondes, qui se propagent à partir du côté de sortie du son vers la zone de focalisation, et **en ce que** la forme de la surface d'émission de ladite unité de focalisation, qui varie de la forme circulaire, et/ou la forme du cône d'émission de ladite unité de focalisation - et ainsi la position focale relative à la surface d'émission de ladite unité de focalisation est adaptée à l'application respective,
et/ou **en ce que** ladite unité génératrice de son comprend au moins un vibrateur à large bande, dont la fréquence d'excitation varie ladite unité de commande pour l'ajustage de l'écart entre ladite zone focale et ladite unité de focalisation.

2. Dispositif selon la revendication 1,
**caractérisé en ce que** ladite unité de focalisation consiste en un matériau, dont l'impédance acoustique se trouve au-dedans de la gamme entre l'impédance de ladite unité génératrice de son et l'impédance du milieu, le long de la voie de propagation de son, en aval de ladite unité de focalisation.

3. Dispositif selon la revendication 2,
**caractérisé en ce que** ladite (lesdits) couche(s) adaptatrice(s), qui est/sont disposée(s) entre la surface d'émission de ladite unité génératrice de son et la surface d'entrée du son de ladite unité de focalisation, a/ont un niveau d'impédance entre le niveau de ladite unité de focalisation et l'impédance de ladite unité génératrice de son.

4. Dispositif selon la revendication 3,
**caractérisé en ce que** lesdites couches adaptatrices sont des couches à basse réflexion.

5. Dispositif selon la revendication 3 or 4,
**caractérisé en ce que** lesdites couches adaptatrices sont des couches à λ/4 au moins en partie.

6. Dispositif selon une quelconque des revendications 1 à 5,
**caractérisé en ce qu'**au moins une couche adaptatrice est appliquée sur une surface de limite de ladite unité génératrice de son, qui est opposée à la surface d'émission.

7. Dispositif selon la revendication 6,
**caractérisé en ce que** ladite couche adaptatrice, qui est appliquée sur la surface de limite opposée, a une impédance similaire à l'impédance de ladite unité génératrice de son ou une impédance nettement différente.

8. Dispositif selon une quelconque des revendications 1 à 7,
**caractérisé en ce que** ladite unité génératrice de son comprend des piézo vibrateurs (2).

9. Dispositif selon une quelconque des revendications 1 à 6,
**caractérisé en ce que** ladite unité de focalisation sert en tant que corps d'appui de l'entière structure.

10. Dispositif selon une quelconque des revendications 1 à 9,
**caractérisé en ce que** ladite unité génératrice de son comprend une pluralité des vibrateurs individuels.

11. Dispositif selon la revendication 10,
**caractérisé en ce que** lesdits vibrateurs individuels sont disposés de la manière d'une mosaïque.

12. Dispositif selon la revendication 11,
**caractérisé en ce que** lesdits vibrateurs individuels ont la forme des anneaux en arrangement concentrique.

13. Dispositif selon la revendication 12,
**caractérisé en ce que** lesdits anneaux sont configurés de la manière des zones du type Fresnel.

14. Dispositif selon une quelconque des revendications 10 to 13,
**caractérisé en ce que** ladite unité de commande sert à commander lesdits vibrateurs individuels séparément.

15. Dispositif selon la revendication 14,
**caractérisé en ce que** ladite unité de commande sert à commander lesdits vibrateurs individuels aux positions de phase différentes.

16. Dispositif selon la revendication 14 or 15,
**caractérisé en ce que** ladite unité de commande sert à commander lesdits vibrateurs individuels aux fréquences différentes.

17. Dispositif selon une quelconque des revendications 1 à 16,
**caractérisé en ce qu'**un moyen de localisation est disposé, qui permet la détection de la zone à traiter, et en particulier les lieux de calculs, des concrétions, etc.

18. Dispositif selon la revendication 17,
**caractérisé en ce que** ledit moyen de localisation est disposé dans une zone centrale de la surface de sortie de son de ladite unité de focalisation.

19. Dispositif selon la revendication 17,
**caractérisé en ce que** ledit moyen de localisation est disposé à l'extérieur de la zone centrale de la surface de sortie de son de ladite unité de focalisation.

20. Dispositif selon une quelconque des revendications 17 à 19,
**caractérisé en ce que** ledit moyen de localisation comprend un moyen de localisation aux rayons roentgen.

21. Dispositif selon une quelconque des revendications 17 à 20,
**caractérisé en ce que** ledit moyen de localisation comprend un moyen de localisation aux ultrasons.

22. Dispositif selon la revendication 21,
**caractérisé en ce que** ledit moyen de localisation est un dispositif aux ultrasons à engendre des images type B.

23. Dispositif selon une quelconque des revendications 1 à 22,
**caractérisé en ce que** ladite surface d'émission de son de ladite structure du type Fresnel a une forme en symétrie de révolution.

24. Dispositif selon une quelconque des revendications 1 à 23,
**caractérisé en ce que** ladite unité de commande varie la fréquence, par laquelle elle commande ladite unité génératrice de son, pour la variation des la position de la focale.

25. Dispositif selon une quelconque des revendications 1 à 24,
**caractérisé en ce qu'**un milieu de couplage est disposé entre la surface d'émission de son de ladite unité de focalisation et la zone de couplage du corps à traiter.

26. Dispositif selon la revendication 25,
**caractérisé en ce que** ledit milieu de couplage est limité par un coussin flexible.

27. Dispositif selon la revendication 25 or 26,
**caractérisé en ce que** ledit milieu de couplage constitue une voie d'avancement flexible.
